Europäisches Patentamt

European Patent Office

Office européen des brevets

(11) Veröffentlichungsnummer: **0 280 846 B1**

# EUROPÄISCHE PATENTSCHRIFT

(45) Veröffentlichungstag der Patentschrift:
06.03.91 Patentblatt 91/10

(51) Int. Cl.$^5$: **A61M 16/14,** A61M 16/18, A61M 16/01

(21) Anmeldenummer: 88100487.3

(22) Anmeldetag: 15.01.88

(54) Vorrichtung zur Steuerung eines Verdunsters mittels Druckschwankungen.

(30) Priorität: 24.01.87 DE 3702136

(43) Veröffentlichungstag der Anmeldung:
07.09.88 Patentblatt 88/36

(45) Bekanntmachung des Hinweises auf die Patenterteilung:
06.03.91 Patentblatt 91/10

(84) Benannte Vertragsstaaten:
DE FR GB SE

(56) Entgegenhaltungen:
EP-A- 0 146 220
DE-B- 1 566 596

(56) Entgegenhaltungen:
DE-B- 2 507 261
GB-A- 961 503
GB-A- 1 104 585
US-A- 2 864 363
US-A- 3 524 463

(73) Patentinhaber: Drägerwerk Aktiengesellschaft
Moislinger Allee 53-55
W-2400 Lübeck 1 (DE)

(72) Erfinder: Heim, Ulrich, Dr. Phys.
Hohelandstrasse 61
W-2400 Lübeck (DE)
Erfinder: Albarda, Scato, Dipl.-Ing.
Am Kannenbruch 11
W-2061 Gross Schenkenberg (DE)

EP 0 280 846 B1

**Beschreibung**

Die Erfindung betrifft eine Vorrichtung zur Erzeugung eines Gasgemisches nach den Merkmalen des Oberbegriffs on Anspruch 1 und 2.

Ein derartiger Verdunster ist aus der DE-OS 25 07 261 bekannt. Er dient zur Verdunstung eines flüssigen Narkosemittels, das in einem entsprechenden Behälter enthalten ist, durch den ein Teilstrom eines Trägergases geleitet wird. Das Trägergas kann zum Beispiel Sauerstoff sein, welcher mit dem über einen Docht verdunsteten Narkosemittel gesättigt wird. Über eine parallel zu dem Eingang und dem Ausgang des Verdunsters geschaltete By-pass-Leitung wird der narkosemittelfreie Teilstrom des Trägergases dem narkosemittelhaltigen wieder zugeführt und diese Gasmischung einem Verbraucher, in dem bekannten Fall ein Patient, zur Atmung zugeführt. Die Konzentration des gebildeten Gasgemisches wird durch das einstellbare Verhältnis der Durchströmung des Verdunsters und der By-pass-Leitung bestimmt.

Da die verdunstete Menge einer Flüssigkeit von mehreren Parametern abhängig ist, wie zum Beispiel der Flüssigkeitstemperatur, ist bei dem bekannten Verdunster eine entsprechende Vorrichtung zur Kompensierung dieses Temperatureinflusses auf die Verdunstungsmenge vorgesehen. Andere äußere, von dem Benutzer des Verdunsters nicht beeinflußbare Parameter können jedoch nach wie vor zu unerwünschten Schwankungen der abgegebenen Verdunstungsmenge führen :

Die Einstellung der Teilströme durch den Verdunster und durch die By-pass-Leitung wird bei dem vorbekannten Verdunster durch einen einstellbaren Strömungswiderstand erzeugt, welcher von der Strömungsgeschwindigkeit abhängig ist. Weiterhin können Druckschwankungen am Ausgang des Verdunsters zu Strömungsschwankungen im Verdunster selbst und damit zu Schwankungen bei der Abgabe der Verdunstungsmenge an das Trägergas führen. Auch bei Änderungen der Gasart des Trägergases ändern sich die Strömungsverhältnisse in den Strömungswiderständen und führen so zu einer unterschiedlichen Aufteilung der Teilströme durch den Verdunster und durch die By-pass-Leitung.

Da ein großer Anwendungsbereich der vorbekannten Verdunster zur Verdunstung flüchtiger Narkosemittel mit hohem Partialdruck zur Beatmung von narkotisierten Patienten dient und weil bei dieser Anwendung eine möglichst genaue und schwankungsfreie Dosierung der Narkosemittel erforderlich ist, sind die genannten äußeren Einflüsse auf den Verdunster erheblich und führen zu unerwünschten Konzentrationsschwankungen von Narkosemitteln im Trägergas.

Aus der GB-A-1 104 585 ist ein Narkosemittelverdunster bekannt, dessen Verdunsterkammer durch ein Eingangsventil von der Versorgungsleitung absperrbar ist, und wobei eine By-Pass-Leitung zur Umgehung der Verdunsterkammer in eine Verbraucherleitung mündet. Der Ausgang der Verdunsterkammer ist mit einem By-Pass-Ventil versehen. Eingangsventil und By-Pass-Ventil sind mit einem Einstellknopf betätigbar. Bei geöffnetem Eingangsventil kann durch weiteres Verstellen des Einstellknopfes mittels eines Kopplungsgestänges das By-Pass-Ventil mehr oder weniger weit geöffnet und auch geschlossen werden. Die Größe des Öffnungsquerschnittes im By-Pass-Ventil bestimmt die Menge an Trägergas, welche in die Verdunsterkammer strömt, das verdunstende Narkosemittel mitreißt und es am Ausgang der Verdunsterkammer dem Trägergas zuführt. Das Verhältnis von narkosemittelfreiem Trägergas durch die By-Pass-Leitung zu dem mit Narkosemittel angereichertem Trägergas durch die Verdunsterkammer bestimmt die Narkosemittelkonzentration in der Verbraucherleitung.

Der vorliegenden Erfindung liegt somit die Aufgabe zugrunde, eine Vorrichtung der genannten Art so zu verbessern, daß der Einfluß gasartspezifischer Parameter auf die Verdunstungsmenge verringert wird.

Die Lösung der Aufgabe erfolgt durch die im kennzeichnenden Teil des Anspruchs 1 oder 2 aufgeführten Merkmale.

Der Vorteil der vorliegenden Erfindung kann im wesentlichen darin gesehen werden, daß durch gewollte Druckerhöhung eine Aufladung der Verdunsterkammer mit dem Trägergas erfolgt und daß durch gesteuerte Abgabe des verdunsteten Gasanteils an das Trägergas die Konzentration in dem Gasgemisch variiert werden kann. Dabei spielen strömungsabhängige und gasartspezifische Parameter keine Rolle mehr.

Wenn bei ausgangsseitiger Unterbrechung der Verdunsterleitung auch gleichzeitig die Verbraucherleitung unterbrochen ist, sind gleichzeitig der By-pass-Strom und der Trägergasstrom unterbrochen, wobei jedoch der Eingang des Verdunsters an die Versorgungsleitung angeschlossen bleibt. Dadurch baut sich innerhalb des Verdunsters ein Druck des Trägergases auf, welcher durch das die Trägergasströmung erzeugende Förderungsmittel, wie zum Beispiel eine pneumatisch oder elektrisch betriebene Dosiereinheit oder Pumpe, bestimmbar ist. Diese Aufladungsperiode wird dadurch beendet, daß gleichzeitig der Verdunster ausgangsseitig sowohl mit der By-pass-Leitung als auch mit der Verbraucherleitung verbunden und eingangsseitig von der Versorgungsleitung getrennt wird. Nunmehr kann durch die By-pass-Leitung das Trägergas zum Verbraucher strömen, wobei durch den Druckabbau in dem Verdunster die Verdunstungsmenge in Richtung Verbraucherleitung abgegeben wird. Durch die wechselweise Beschaltung von Verdunstereingang beziehungsweise -ausgang wird ein pulsweises Aufladen des Verdunsters mit Trägergas zu dessen Anreicherung mit Verdunstungsmittel verwirklicht, welches gezwungenermaßen nur in Richtung der Verbraucherleitung aus ihm entweichen kann. Eine Pendelströmung des Trägergases durch den Verdunster wird dadurch vermieden.

Da die zur Aufladung des Verdunstervolumens erforderlichen Drücke sehr gering sind im Vergleich zu den Drücken, welche zur Erzeugung einer Trägergasströmung notwendig sind, wird bei der Absperrung des Verdunsters von der Verbraucher- und der By-pass-Leitung stets ein genügend hoher Druck aufgebaut, so daß sich eine ausreichende Menge an gasförmigem Verdunstermedium bilden kann.

Mit jedem Zyklus der Unterbrechung und Verbindung des Verdunsters mit dem Trägergasstrom wird eine definierte Menge an gesättigtem Trägergas in die Verbraucherleitung geleitet, welche durch Wahl der Verbindungs- und Unterbrechungszeiten variiert werden kann.

Die Unterbrechung des Trägergasstromes erfolgt durch ein ansteuerbares Eingangs- und Ausgangsventil jeweils am Eingang und Ausgang des Verdunsters. Die Unterbrechung der Verbraucherleitung nimmt ein Sperrventil vor, wobei gleichzeitig der Ausgang des Verdunsters durch das Ausgangsventil von der By-pass-Leitung und der Verbraucherleitung getrennt ist. Wird das Absperrventil und das Ausgangsventil geöffnet und das Eingangsventil geschlossen, kann der Trägergasstrom aus der By-pass-Leitung, angereichert mit dem verdunsteten Medium, durch die Verbraucherleitung zum Verbraucher geführt werden, wobei eine Rückströmung aus dem Verdunster in seine Eingangsleitung durch das geschlossene Eingangsventil verhindert wird. Die Konzentration des Gasgemisches in der Verbraucherleitung wird durch die Öffnungsdauer des Absperrventils bestimmt, indem dadurch die von der Verdunstungskammer abgegebene Gasmenge mit einer so festgelegten Trägergasmenge in der Verbraucherleitung verdünnt wird. Die Steuerung der Ventile kann entweder durch Pulsbreitenmodulation oder Pulsfrequenzmodulation erfolgen. Dabei sind typische Werte für einen vollständigen Schaltzyklus etwa 50 ms, wobei die Zeit für das Aufladen des Verdunsters mit dem Trägergasdruck etwa 1 ms dauert.

Wenn auch durch das günstige Pulsbreitenverhältnis die Druckschwankungen in der Verbraucherleitung gering sind, kann es vorteilhaft sein, zur weiteren Glättung der Druckschwankungen und zum Ausgleich von Konzentrationsspitzen an Verdunstungsmedium im Trägergas einen Pufferraum in der Verbraucherleitung strömungsabwärts zum Absperrventil vorzusehen.

Die Vorrichtung ist besonders geeignet, um in Verbindung mit einer genauen Dosiereinheit das Verdunstungsmedium in kleinsten Mengen und reproduzierbar in den Trägergasstrom einzuleiten.

Durch die Zusammenfassung des in der Verdunsterleitung angeordneten Eingangs- und Ausgangsventils zu einem einzigen Magnetventil mit zwei in den vom Trägergas durchspülbaren Ventilraum mündenden Sitzen, die die Enden der Verdunsterleitung bilden, und Führung der By-pass-Leitung diametral durch den Ventilraum wird nicht nur eine sehr kompakte Bauform erzielt, sondern vor allem jeder Totraum zwischen Ventilsitz und By-pass-Leitung vermieden, der zu Verfälschungen der Konzentration des Gasgemisches führen könnte. Bei Verwendung einer zwischen den Sitzen schaltbaren Kugel als Verschlußkörper läßt sich eine hohe Schaltfrequenz verwirklichen.

Ein Ausführungsbeispiel der Erfindung ist anhand einer schematischen Zeichnung dargestellt und wird im folgenden näher erläutert.

Es zeigen

Fig. 1 ein Blockdiagramm für die Beschaltung eines Verdunsters,
Fig. 2 ein Blockdiagramm für eine weitere Beschaltungsmöglichkeit eines Verdunsters,
Fig. 3 einen Schnitt durch das Kugel-Magnetventil.

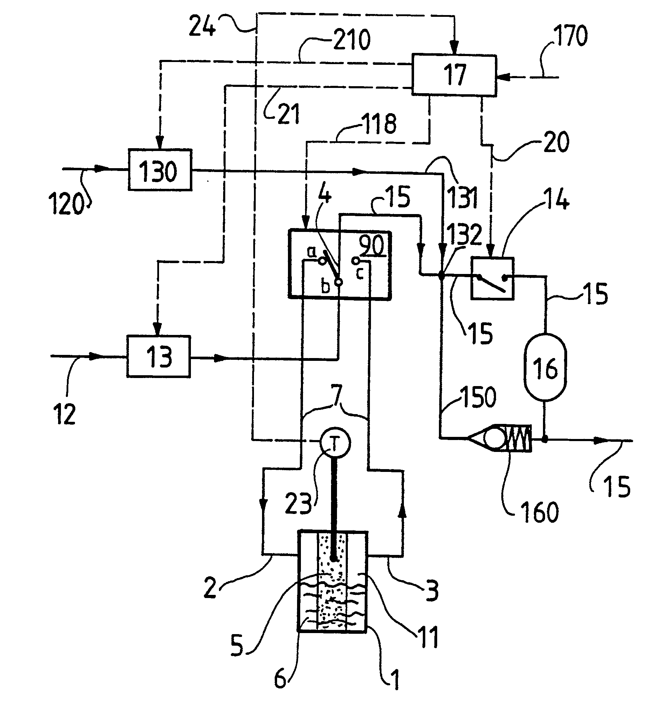

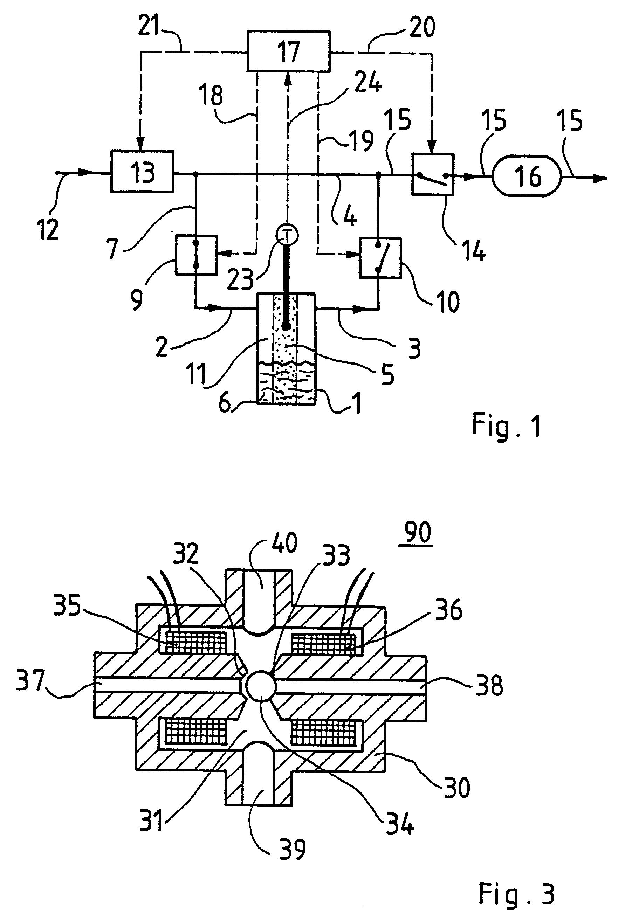

Der in Figur 1 dargestellte Verdunster (1) ist mit seinem Eingang (2) und seinem Ausgang (3) über eine Verdunsterleitung (7) an eine Umgehungsbeziehungsweise By-pass-Leitung (4) angeschlossen. In dem Verdunster (1) ist ein Docht (5) in eine Verdunstungsflüssigkeit (6), zum Beispiel ein Narkosemittel, eingetaucht. Sowohl dem Eingang (2) als auch dem Ausgang (3) des Verdunsters (1) sind ein steuerbares Eingangsventil (9) und ein steuerbares Ausgangsventil (10) vor- beziehungsweise nachgeschaltet. Das Trägergas, welches zum Beispiel Sauerstoff sein kann, wird von einer Versorgungsleitung (12) in eine Dosiereinheit (13) und von dort in die Verdunsterleitung (7) und die By-pass-Leitung (4) geleitet. Die By-pass-Leitung (4) führt weiter in eine Verbraucherleitung (15), und diese wiederum über ein Absperrventil (14) und einen Pufferbehälter (16) zu einem nicht dargestellten Verbraucher. Die Steuerung der Ventile (9, 10, 14) übernimmt eine Steuereinheit (17) über die jeweiligen Steuerleitungen (18, 19, 20). Weiterhin gibt die Steuereinheit (17) Steuerbefehle über eine Dosiersteuerleitung (21) an die Dosiereinheit (13) ab. Ein Temperaturfühler (23) im Verdunster (1) übermittelt über eine Temperatursignalleitung (24) die entsprechenden Temperaturwerte an die Steuereinheit (17).

In dem dargestellten Schaltzustand sind das Eingangsventil (9) geöffnet, das Ausgangsventil (10) und das Absperrventil (14) geschlossen. Als Beispiel wird angenommen, daß das Trägergas Sauerstoff und die Verdunstungsflüssigkeit (6) ein Narkosemittel ist. Von der Dosiereinheit (13) wird Sauerstoff in die Verdunstungskammer (11) des Verdunsters (1) geleitet und baut dort einen Überdruck auf. Aus dem Narkosemittel (6) verdunstet Narkoseflüssigkeit in das unter leichtem Überdruck befindliche Sauerstoffgas, und zwar maximal bis zur Sättigung der Gasphase bei der herrschenden Temperatur. Die Steuereinheit (17) gibt an die Ventile (9, 10, 14) über die entsprechenden Steuerleitungen (18, 19, 20) einen Steuerbefehl derart, daß das Eingangsventil (9) geschlossen und damit der Aufladungsvorgang zur Erzeugung eines Überdruckes abgebrochen wird, und daß gleichzeitig das Ausgangsventil (10) und das Absperrventil (14) geöffnet werden. Damit tritt unmittelbar die Strömung des Trägergases über die By-pass-Leitung (4) in die Verbraucherleitung (15) mit Absperrventil (14) und Pufferbehälter (16) ein. Gleichzeitig strömt das mit Narkosemittel angereicherte Trägergas aus dem Verdunster (1) über das Ausgangsventil (10) in die By-pass-Leitung (4) zur Verbraucherleitung (15), wodurch der Überdruck auch in der Verdunstungskammer (11) abgebaut wird. Somit erhält der nicht dargestellte Verbraucher ein mit Narkosemittel angereichertes Sauerstoffgasgemisch. Durch Variation der Schaltzeiten des Absperrventils (14) kann die Konzentration an Narkosemittel bestimmt werden, welche dem Verbrauchergas während eines Schaltzyklus zugeführt werden soll.

In Figur 2 ist eine weitere Beschaltungsmöglichkeit des Verdunsters (1) angegeben. Dabei sind gleichfalls in Figur 1 dargestellte Bauteile mit den gleichen Bezugsziffern bezeichnet. Das Trägergas, beispielsweise wiederum Sauerstoff, wird über die Dosiereinheit (13) einem Schaltventil (90) zugeführt, welches als ein Umschalter mit zwei Arbeitspositionen und einem ständigen Durchgang als By-pass-Leitung ausgebildet ist. Dabei ist der Mittenanschluß (b) in der dargestellten Stellung mit dem Seitenanschluß (a) verbunden, wodurch der Eingang (2) des Verdunsters (1) an den Trägergasstrom aus der Versorgungsleitung (12) angeschlossen ist. Der Ausgang (3) des Verdunsters (1) ist über den geschlossenen Seitenanschluß (c) von der By-pass-Leitung (4) abgetrennt. Die By-pass-Leitung (4) ist mit der Verbraucherleitung (15) verbunden. Eine zusätzliche Gasleitung (131) mündet in die Verbraucherleitung (15) an einem Sammelpunkt (132), der einerseits mit dem Absperrventil (14) und andererseits über eine Umgehungsleitung (150) an ein Überdruckventil (160) verbunden ist. In der dargestellten Schaltstellung unterbricht das Absperrventil (14) die Verbraucherleitung (15). Eine weitere Versorgungsleitung (120) ist über eine Zusatzdosiereinheit (130) mit der Gasleitung (131) verbunden. Mit diesen Geräteteilen kann beispielsweise ein weiteres, gasförmiges Narkosemittel zu dem Sammelpunkt (132) geleitet werden. Die Zusatzdosiereinheit (130) ist über ihre Steuerleitung (210) ebenfalls an die Steuereinheit (17) angeschlossen. Von dieser sind weiterhin, entsprechend den über eine Eingabeleitung (170) eingegebenen Sollwertdaten, die Dosiereinheit (13), der Umschalter (90) über eine Umschalterleitung (118) sowie das Absperrventil (14) ansteuerbar.

In dem dargestellten Schaltungszustand wird die Verdunstungskammer (11) mit dem Trägergas, beispielsweise Sauerstoff aufgeladen, wobei der Ausgang (3) des Verdunsters (1) über den geschlossenen Seitenanschluß (c) des Umschalters (90) von der By-pass-Leitung (4) getrennt und die Versorgungsleitung (15) wegen des geschlossenen Absperrventils (14) unterbrochen ist. Das Überdruckventil (160) in der Umgehungsleitung (150) sorgt dafür, daß bei unerwünscht hoher Druckerzeugung entweder in der By-pass-Leitung (4) oder in der Gasleitung (131) eine Druckentlastung in die Verbraucherleitung (15) und den Pufferbehälter (16) möglich ist. Nach Vorgabe durch die Steuereinheit (17) werden der Umschalter (90) und das Absperrventil (14) in die nicht dargestellte, zweite Schaltstellung gebracht, bei welcher der Mittenanschluß (b) mit dem Seitenanschluß (c) verbunden beziehungsweise das Absperrventil (14) geöffnet ist. Das mit Narkosemittel angereicherte Gas aus der Verdunstungskammer (11) kann nunmehr in die By-pass-Leitung (4) und die Verbraucherleitung (15) strömen.

In Fig. 3 ist die Ausbildung des Umschalters (90) als Magnetventil gezeigt, seine Beschaltung ist sinngemäß Fig. 2 zu entnehmen. Ein Gehäuse (30) bildet im Inneren einen Ventilraum (31) mit zwei einander gegenüberliegenden Sitzen (32, 33), zwischen denen eine Kugel (34) spielen kann. Jeder Sitz (32, 33) ist von einer Spule (35, 36) umgeben. Zu den Sitzen (32, 33) führen Verbindungen (37, 38), die mit dem Eingang (2) bzw. dem Ausgang (3) des Verdunsters (1) verbunden sind. Der Ventilraum (31) besitzt in einer zwischen den Sitzen (32, 33) und senkrecht zu deren Achse verlaufenden Ebene zwei einander gegenüberliegende Anschlüsse (39, 40), deren einer (39) mit der Versorgungsleitung (12) und deren anderer (40) mit der Verbraucherleitung (15) verbunden ist. Durch Ansteuerung von der Steuereinheit (17) werden die Spulen (35, 36) wechselweise erregt. Die Kugel (34) bewegt sich dann auf die jeweils erregte Spule (z. B. 36) zu und verschließt den entsprechenden Sitz (z. B. 33), während der andere (z. B. 32) offen ist. Dabei bildet der Ventilraum (31) im Bereich der Sitze die ständige Verbindung zwischen Versorgungsleitung (12) und Verbraucherleitung (15) und ist je nach Schaltzustand mit dem Eingang (2) oder Ausgang (3) des Verdunsters (1) verbunden; er entspricht somit einer aufs äußerste verkürzten By-pass-Leitung (4). Toträume zwischen der Schließlinie der Kugel (34) auf den Sitzen (32 bzw. 33) und der so gebildeten By-pass-Leitung gibt es damit nicht.

## Ansprüche

1. Vorrichtung zur Erzeugung eines Gasgemisches, bei der ein Verdunster (1) in eine Verdunsterleitung (7) eingeschaltet ist, die von einer Versorgungsleitung (12) ausgeht und in eine Verbraucherleitung (15) mündet, wobei die Verdunsterleitung (7) durch eine By-Pass-Leitung (4) überbrückt ist, und sowohl der Eingang (2) als auch der Ausgang (3) der Verdunsterkammer (11) durch ein Eingangsventil (9) und ein Ausgangsventil (10) von der By-Pass-Leitung (4) absperrbar sind, dadurch gekennzeichnet, daß eine Steuereinheit (17) sowohl die Ventile (9, 10) als auch ein in die Verbraucherleitung (15) eingefügtes Absperrventil (14) derart betätigt, daß bei die Verbindung zwischen By-Pass-Leitung (4) und Verdunsterkammer (11) herstellenden geöffnetem Eingangsventil (9) sowohl das Ausgangsventil (10) als auch das Absperrventil (14) zur Erzeugung eines in der Verdunsterkammer (11) herrschenden Druckes geschlossen sind, und daß bei geschlossenem Eingangsventil (9) das Ausgangsventil (10) und das Absperrventil (14) zur Dosierung des mit dem in der Verdunsterkammer (11) befindlichen Narkosemittel (6) angereicherten Trägergases geöffnet sind.

2. Vorrichtung zur Erzeugung eines Gasgemisches, bei der ein Verdunster (1) in eine Verdunsterleitung (7) eingeschaltet ist, die von einer Versorgungsleitung (12) abzweigt, von dort zum Eingang (2) der Verdunsterkammer (11) führt und über deren Ausgang (3) in eine Verbraucherleitung (15) mündet, wobei sowohl der Eingang (2) durch ein Eingangsventil (90a, b) von der Versorgungsleitung (12) als auch der Ausgang (3) der Verdunsterkammer (11) durch ein Ausgangsventil (90b, c) von der Versorgungsleitung (15) absperrbar sind, dadurch gekennzeichnet, daß eine Steuereinheit (17) sowohl die Ventile (90a, b, c) als auch ein in die Verbraucherleitung (15) eingefügtes Absperrventil (14) derart betätigt, daß bei die Verbindung der Versorgungsleitung (12) mit dem Eingang (2) herstellenden geöffnetem Eingangsventil (90a, b) sowohl das Ausgangsventil (90b, c) als auch das Absperrventil (14) zur Erzeugung eines in der Verdunsterkammer (11) herrschenden Druckes geschlossen sind, und daß bei geschlossenem Eingangsventil (90a, b) das Ausgangsventil (90b, c) und das Absperrventil (14) zur Dosierung des mit dem in der Verdunsterkammer (11) befindlichen Narkosemittels (6) angereicherten Trägergases geöffnet sind.

3. Vorrichtung nach Anspruch 1 oder 2, dadurch gekennzeichnet, daß in der Verbraucherleitung (15) strömungsabwärts zum Absperrventil (14) ein Pufferraum (16) vorgesehen ist.

4. Vorrichtung nach einem der Ansprüche 1 bis 3, dadurch gekennzeichnet, daß in der Versorgungsleitung (12) eine Dosiereinheit (13) vorgesehen ist.

5. Vorrichtung nach Anspruch 2, dadurch gekennzeichnet, daß das ansteuerbare Eingangs- und Ausgangsventil (90) als Magnetventil mit zwei in den vom Trägergas durchspülbaren Ventilraum (31) mündenden, mit Eingang (2) und Ausgang (3) des Verdunsters (1) verbundenen Sitzen (32, 33) ausgebildet und mit zwei einander gegenüberliegenden, zum Ventilraum (31) führenden Anschlüssen (39, 40) zwischen Versorgungsleitung (12) und Verbraucherleitung (15) eingefügt ist.

## Claims

1. Device for the production of a gas mixture, in which a vaporizer (1) is inserted into a vaporizing line (7) which emanates from a supply line (12) and opens into a user line (15), whereby the vaporizing line (7) is bridged by a by-pass line (4), and both the inlet (2) and the outlet (3) of the vaporizing chamber (11) can be closed by an inlet valve (9) and an outlet valve (10) from the by-pass line (4), characterized in that a control unit (17) actuates both the valves (9, 10) and a closing valve (14) inserted into the user line (15) in such a way that with the inlet valve opened (9) establishing a connection between by-pass line (4) and vaporizing chamber (11) both the outlet valve (10) and the closing valve (14) are closed for the production of a pressure prevailing in the evaporator chamber (11), and in that with the inlet valve closed (9) the outlet valve (10) and the closing valve (14) are opened for the dosing of the carrier gas enriched with the narcotic (6) located in the vaporizing chamber (11).

2. Device for the production of a gas mixture, in which a vaporizer (1) is inserted into a vaporizing line (7), which branches off from a supply line (12), leads from there to the inlet (2) of the vaporizing chamber (11) and opens by means of its outlet (3) into a user line (15), whereby both the inlet (2) can be closed by an inlet valve (90a, b) from the supply line (12) and the outlet (3) of the vaporizing chamber (11) can be closed by an outlet valve (90b, c) from the supply line (15), characterized in that a control unit (17) actuates both the valves (90a, b, c) and a closing valve (14) inserted into the user line (15) in such a way that with the inlet valve (90a, b) opened establishing a connection of the supply line (12) to the inlet (2), both the outlet valve (90b, c) and the closing valve (14) are closed for the production of a pressure prevailing in the vaporizing chamber (11), and in that with closed inlet valve (90a, b) the outlet valve (90b, c) and the closing valve (14) are opened for the dosing of the carrier gas enriched with the narcotic (6) located in the vaporizing chamber (11).

3. Device according to claim 1 or 2, characterized in that a buffer space (16) is provided in the user line (15) downstream of the closing valve (14).

4. Device according to one of claims 1 to 3, characterized in that a dosing unit (13) is provided in the supply line (12).

5. Device according to claim 2, characterized in that the controllable inlet and outlet valve (90) is formed as solenoid valve with two seats (32, 33) opening into the valve space (31), which can be rinsed by the carrier gas, and connected to the inlet (2) and outlet (3) of the vaporizer (1), and is inserted with two connections (39, 40) lying opposite each other and leading to the valve space (31) between supply line (12) and user line (15).

## Revendications

1. Dispositif destiné à produire un mélange de gaz dans lequel un évaporateur (1) est monté dans un conduit d'évaporateur (7) qui part d'un conduit d'alimentation (12) et débouche dans un conduit de récepteur (15), le conduit d'évaporateur (7) étant ponté par un conduit de dérivation (4) et l'entrée (2) comme la sortie (3) de la chambre d'évaporateur (11) pouvant être séparées du conduit de dérivation (4) par une vanne d'entrée (9) et par une vanne de sortie (10), caractérisé en ce qu'une unité de commande (17) actionne les vannes (9, 10) de même qu'une vanne d'arrêt (14) insérée dans le conduit de récepteur (15), de manière que lorsque la vanne d'entrée (9) faisant communiquer le conduit de dérivation (4) et la chambre d'évaporateur (11) est ouverte, la vanne de sortie (10) et la vanne d'arrêt (14) sont fermées pour produire une pression dans la chambre d'évaporateur (11) et en ce que, lorsque la vanne d'entrée (9) est, fermée, la vanne de sortie (10) et la vanne d'arrêt (14) sont ouvertes pour doser le gaz de support enrichi avec le narcotique (6) se trouvant dans la chambre d'évaporateur (11).

2. Dispositif destiné à produire un mélange de gaz dans lequel un évaporateur (1) est monté dans un conduit d'évaporateur (7) qui part d'un conduit d'alimentation (12), de là, mène à l'entrée (2) de la chambre d'évaporateur (11) et débouche, par sa sortie (3), dans un conduit de récepteur (15), l'entrée (2) pouvant être séparée par une vanne d'entrée (90a, b) du conduit d'alimentation (12) et la sortie (3) de la chambre d'évaporateur (11) pouvant être séparée par une vanne de sortie (90b, c) du conduit d'alimentation (15), caractérisé en ce qu'une unité de commande (17) actionne les vannes (90a, b, c) ainsi qu'une vanne d'arrêt (14), insérée dans le conduit de récepteur (15), de manière que lorsque la vanne d'entrée (90a, b), faisant communiquer le conduit d'alimentation (12) et l'entrée (2), est ouverte, la vanne de sortie (90b, c) ainsi que la vanne d'arrêt (14) sont fermées, pour produire une pression dans la chambre d'évaporateur (11) et en ce que, lorsque la vanne d'entrée (90a, b) est fermée, la vanne de sortie (90b, c) et la vanne d'arrêt (14) sont ouvertes pour doser le gaz de support enrichi avec le narcotique (6) se trouvant dans la chambre d'évaporateur (11).

3. Dispositif selon la revendication 1 ou 2, caractérisé en ce qu'on prévoit un compartiment-tampon (16) dans le conduit de récepteur (15), en aval de la vanne d'arrêt (14).

4. Dispositif selon l'une des revendications 1 à 3, caractérisé en ce qu'on prévoit une unité de dosage (13) dans le conduit d'alimentation (12).

5. Dispositif selon la revendication 2, caractérisé en ce que la vanne d'entrée et de sortie (90) commandable est une électrovanne comportant deux sièges (32, 33) débouchant dans la chambre de vanne (31), traversée par le gaz de support, reliés à l'entrée (2) et à la sortie (3) de l'évaporateur (1) et est insérée entre le conduit d'alimentation (12) et le conduit de récepteur (15), avec deux raccords (39, 40) opposés, menant à la chambre de vanne (31).

EP 0 280 846 B1

Fig. 2

7

Fig . 1

Fig . 3